# EUROPEAN PATENT SPECIFICATION

(11) **EP 1 979 013 B1**
(45) Date of publication and mention of the grant of the patent: **09.03.2011**
(21) Application number: 07718018.0
(22) Date of filing: 11.01.2007
(51) Int. Cl.: A61L 27/34, A61L 27/54, A61L 31/10, A61L 31/16, A61L 29/16, A61L 29/08

(54) **COATED MEDICAL DEVICES AND METHODS OF MAKING THE SAME**
BESCHICHTETE MEDIZINISCHE VORRICHTUNGEN UND VERFAHREN ZU IHRER HERSTELLUNG
DISPOSITIFS MÉDICAUX À ENROBAGE ET PROCÉDÉS DE FABRICATION DE CEUX-CI

(30) Priority: 12.01.2006 US 332606
(43) Date of publication of application: 15.10.2008
(73) Proprietor: Boston Scientific Limited, Christ Church (BB)
(72) Inventor: JANG, Eun-Hyun, Allston, MA 02134 (US)
(74) Representative: Peterreins, Frank
(86) International application number: PCT/US2007/000814
(87) International publication number: WO 2007/084361

(56) References cited:
- EP-A1- 1 260 214
- WO-A-01/01890
- WO-A-01/56646
- WO-A-01/87375
- WO-A-95/08305
- US-A- 5 810 786
- US-A- 6 042 875

## Description

### 1. FIELD OF THE INVENTION

The invention relates generally to medical devices that are useful for delivering a biologically active material to a body tissue, such as a body lumen, and methods for making such medical devices. In particular, the invention is directed to a medical device having a surface coated with a coating comprising one or more coating layers. Each coating layer preferably comprises one or more biologically active material that is present in at least two different forms. More particularly, the invention is directed to a medical device having a surface coated with a coating composition that comprises a biologically active material that is present in a first form and a second form. The coating is capable of releasing the two forms of the biologically active material over a time period at different rates. Preferably, the biologically active material inhibits cell proliferation, contraction, migration or hyperactivity (*e.g*., paclitaxel) and/or has anticancer effects (*e.g*., halofuginone). Methods of using the coated medical device for treating or preventing cancer, stenosis and/or restenosis in a subject, preferably a human, are also provided.

### 2. BACKGROUND OF THE INVENTION

Medical devices, such as implantable stents, have been used for delivering biologically active material to body tissue such as a body lumen. These medical devices have been coated with compositions that comprises biologically active material by various methods. For example, spraying is a common technique for applying a coating uniformly to a surface of a medical device, such as a stent. Direct deposition is another method that involves depositing a bead of material along the struts of a stent.

However, many methods for coating medical devices are often inefficient because the surfaces of the medical devices tend to be hydrophobic while many biologically active material that are in an aqueous solution, have a low affinity for the relatively hydrophobic surface. Because of the surface tension between the hydrophobic surface and the aqueous solution of biologically active material, it is often difficult to sufficiently adhere the biologically active material to a medical device surface. The aqueous solution containing the biologically active material does not adequately wet the surface of the medical device. For example, material applied by spraying or direct deposition does not adequately wet the surface of the stent and thus does not remain on the surface.

These coating techniques are also economically inefficient. Large quantities of costly biologically active material are often wasted because it is difficult to adhere them to the surface of the medical device. The high cost coupled with the inefficiency of the coating methods make these existing methods for coating medical devices problematic. Furthermore, because it is difficult to sufficiently adhere the biologically active material to the medical device, it is also difficult to effectively deliver a biologically active material from a medical device to targeted body tissue.

Accordingly, there is a need for a more efficient method of delivering a biologically active material to a targeted body tissue. There is also a need for an efficient method of applying costly biologically active material to a medical device surface, and for such method that will not adversely affect the formulation comprising the biologically active material that is to be applied to the medical device. There is also need a for a medical device made by such methods.

US 5,810,786 describes a drug delivery system comprising propathenone or lidocaine dissolved in thermoplastic resin useful for prolonged release of antiahrrythmia medications. Extrusion, coextrusion, coating and diffusion techniques to form orally digestible granules, fibers, films and tubes such as endotracheal tubes, drainage tubes and other medical devices with the material containing drug are disclosed.

US 6,042,875 and EP 1 260 214 describe medical devices having a drug-releasing coating and methods for making such coated devices. The coating permits timed or prolonged pharmacological activity on the surface of medical devices through a reservoir concept. Specifically, the coating comprises at least two layers: an outer layer containing at least one drugionic surfactant complex overlying a reservoir layer containing a polymer and the drug which is substantially free of an ionic surfactant. Upon exposure to body tissue of a medical device covered with such coating, the ionically bound drug in the outer layer is released into body fluid or tissue. Following release of such bound drug, the ionic surfactant binding sites in the outer layer are left vacant. To maintain the pharmacological activity after delivery of the ionically bound drug, additional amounts of the drug are incorporated in the reservoir layer in a manner which allows the drug, which is substantially free of ionic surfactants, to complex with the vacant binding sites of the ionic surfactant of the outer layer.

WO 01/087375 describes an intraluminal medical device comprising drugs, agents or compounds to be utilized in the treatment of vascular disease. In particular, the intraluminal medical device comprises a stent having a substantially tubular structure having an inner surface and an outer surface; a layer of one or more antiproliferative compounds is affixed to the outer surface of the tubular structure, a first layer of one or more anti-coagulant compounds is affixed to the inner surface of the tubular structure, and a second layer of one or more anti-coagulant compounds is affixed to the layer of one or more anti-proliferative compounds affixed to the outer surface of the tubular structure.

WO 01156646 describes a medical device adapted for insertion into a human or animal body, characterized in that its exterior surface is coated with i) an inner first layer of a biocompatible carrier providing sustained release of a biologically active agent dissolved or dispersed therein; ii) an outer second layer consisting of a film of said biologically active agent applied on said inner first layer, where said film optionally may contain at least one non-polymeric adjuvant, diluent or carrier.

### 3. SUMMARY OF THE INVENTION

The invention relates generally to drug-eluting medical devices comprising a surface and a coating disposed on at least a portion of the surface as disclosed in any one of the claims. In certain embodiments, the invention relates to medical devices comprising a coating that comprises a biologically active material that is present in two or more forms. In specific embodiments, the invention relates to medical devices comprising a coating that comprises a biologically active material that is present in a first form and a second form. As used herein, the terms "a first form" and "a second form" of a biologically active material refer to the same biologically active material at different physical or chemical states. For example, the first and second form of a biologically active material can include: a dispersion form or a solution form of the biologically active material; a hydrophilic form or a hydrophobic form of the biologically active material; a water soluble form or a water insoluble form of the biologically active material; a lipid soluble form or a lipid insoluble form of the biologically active material; a free acid form or a free base form or a salt form of the biologically active material; an ionized form or a non-ionized form of the biologically active material. The different forms of the biologically active material can occur naturally or be synthesized by any means known to one skilled in the art.

In certain embodiments, the medical device comprises a coating that comprises a first polymer, a first form of a biologically active material, and a second form of the same biologically active material. In one embodiment, the medical device comprises a coating that comprises a first polymer, a dispersion form of a biologically active material, and a solution form of the same biologically active material. In another embodiment, the medical device comprises a coating that comprises a first polymer, a hydrophilic form of a biologically active material, and a hydrophobic form of the same biologically active material. In another embodiment, the medical device comprises a coating that comprises a first polymer, a water soluble form of a biologically active material, and a water insoluble form of the same biologically active material. In another embodiment, the medical device comprises a coating that comprises a first polymer, a free acid form or a free base form of a biologically active material, and a salt form of the same biologically active material. In another embodiment, the medical device comprises a coating that comprises a first polymer, an ionized form of a biologically active material, and a non-ionized form of the same biologically active material.

In certain embodiments, the medical device comprises a coating that comprises a plurality of coating layers. The one or more coating layers may be layered completely or partially on top of each other or disposed on different parts of a surface of the medical device.

In specific embodiments, the medical device comprises a coating that comprises (i) a first coating layer comprising a first polymer and a first form of a biologically active material, and (i) a second coating layer comprising a second polymer and a second form of the same biologically active material. In one embodiment, the medical device comprises a coating that comprises (i) a first coating layer comprising a first polymer and a dispersion form of a biologically active material, and (i) a second coating layer comprising a second polymer and a solution form of the same biologically active material. In another embodiment, the medical device comprises a coating that comprises (i) a first coating layer comprising a first polymer and a hydrophilic form of a biologically active material, and (i) a second coating layer comprising a second polymer and a hydrophobic form of the same biologically active material. In another embodiment, the medical device comprises a coating that comprises (i) a first coating layer comprising a firm polymer and a water soluble form of a biologically active material, and (i) a second coating layer comprising a second polymer and a water insoluble form of the same biologically active material. In another embodiment, the medical device comprises a coating that comprises (i) a first coating layer comprising a firm polymer and a free acid form or a free base form of a biologically active material, and (i) a second coating layer comprising a second polymer and a salt form of the same biologically active material. In another embodiment, the medical, device comprises a coating that comprises (i) a first coating layer comprising a firm polymer and an ionized form of a biologically active material, and (i) a second coating layer comprising a second polymer and a non-ionized form of the same biologically active material.

In certain embodiments, the coating comprises about the same amount or ratio of the different forms of the biologically active material. In certain other embodiments, the coating comprises different amounts or different ratios of the different forms of the biologically active material. In one embodiment, the coating comprises a first form of the biologically active material in a first amount and a second form of the biologically active material in a second amount, wherein the first amount and the second amount are different. In specific embodiments, the first amount is about one hundred times, about fifty times, about thirty times, about twenty times, about ten times, about five times or about two times greater than the second amount. In specific embodiments, the first amount and the second amount are present at a ratio of about 99:1, 95:5, 90:10, 80:20, 70:30, 60:40 or 50:50.

In one embodiment, the coating is capable of providing sustained release of the biologically active material over a time period. The time period for release of the biologically active material from the coating ranges from about 30 minutes, about 1 hour, about 2 hours, about 3 hours, about 4 hours, about 5 hours, about 6 hours, about 12 hours, about 1 day, about 2 days, about 3 days, about 4 days, about 5 days, about 6 days, about 1 week, about 2 weeks, about 3 weeks, about 1 month, about 2 months, about 3 months, about 4 months, about 5 months, about 6 months, about 1 year, about 2 years, or longer. Preferably, the time period for release of the biologically active material from the coating ranges from about 1 hour to about 24 months, preferably, from about 8 hours to about 9 months.

In certain embodiments, the coating is capable of releasing the different forms of the biologically active material in about the same amount In certain other embodiments, the coating is capable of releasing the different forms of the biologically active material in different amounts. In one embodiment, the coating is capable of releasing a first form of the biologically active material in a first amount and a second form of the biologically active material in a second amount, wherein the first amount and the second amount are different. In specific embodiments, the first amount is about one hundred times, about fifty times, about thirty times, about twenty times, about ten times, about five times or about two times greater than the second amount. In specific embodiments, the first amount and the second amount are present at a ratio of about 99:1, 95:5, 90:10, 80:20, 70:30, 60:40 or 50:50.

The coating is capable of releasing the different forms of the biologically active material at different rates. In one embodiment, the coating is capable of releasing a first form of the biologically active material at a first rate and a second form of the biologically active material at a second rate, wherein the first rate and the second rate are different. In specific embodiments, the first rate is about one hundred times, about fifty times, about thirty times, about twenty times, about ten times, about five times or about two times faster than the second rate.

Preferably, the biologically active material inhibits cell proliferation, contraction, migration or hyperactivity. In one embodiment, the biologically active material comprises an immunosuppressant, an antiproliferative agent, or a combination thereof. In a preferred embodiment, the biologically active material comprises an immunosuppressant such as sirolimus, everolimus, tacrolimus, pimecrolimus, or a combination thereof. In another preferred embodiment, the biologically active material comprises an antiproliferative agent such as paclitaxel, an analog thereof, a derivative thereof, or a combination thereof. In another preferred embodiment, the biologically active material comprises halofuginone or a salt form of halofuginone. In certain embodiments, the biologically active material comprises excipients. In certain embodiments, the biologically active material does not comprise excipients.

In one embodiment, the biologically active material is dispersed in the coating. In a preferred embodiment, the biologically active material is uniformly dispersed in the coating.

In certain embodiments, the coating of the medical device comprise one, two, three, four, five or more polymer. In certain embodiments, the coating comprises two, three, four, five or more forms of the biologically active material.

In certain embodiments, the coating comprises a plurality of coating layers. In a specific embodiment, one or more of the different coating layers comprise different forms of the biologically active material. In another specific embodiment, one or more of the different coating layers comprise the same form of the biologically active material.

In one embodiment, the coating comprises (i) a first coating layer that comprises a first form of the biologically active material, and (ii) a second coating layer that comprises a second form of the biologically active material. In a preferred embodiment, the first coating layer is substantially free of the second form of the biologically active material. In another preferred embodiment, the second coating layer is substantially free of the first form of the biologically active material. In yet another preferred embodiment, the first coating layer is substantially free of the second form of the biologically active material, and the second coating layer is substantially free of the first form of the biologically active material.

In certain embodiments, the first coating layer further comprises a first polymer and the second coating layer further comprises a second polymer. In one embodiment, the first coating layer and the second coating layer comprise different polymers or combinations of polymers. In another embodiment, the first coating layer and the second coating layer comprise the same polymer or combinations of polymers.

The polymer can comprise one or more of the following polymers: styrene-isobutylene-styrene, polyurethanes, silicones, polyesters, polyolefins, polyisobutylene, ethylene-alphaolefin copolymers, acrylic polymers and copolymers, vinyl halide polymers, polyvinyl ethers, polyvinylidene halides, polyacrylonitrile, polyvinyl ketones, polyvinyl aromatics, polyvinyl esters, copolymers of vinyl monomers, copolymers of vinyl monomers and olefins, polyamides, alkyd resins, polycarbonates, polyoxymethylenes, polyimides, polyethers, epoxy resins, polyurethanes, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid, polyglycolic acid, polylactic acid-polyethylene oxide copolymers, EPDM rubbers, fluorosilicones, polyethylene glycol, polysaccharides, phospholipids, or a combination thereof.

In a specific embodiment, the coating comprises a hydrophilic polymer. In a more specific embodiment, the hydrophilic polymer comprises polyvinyl alcohol (PVA), poly(L-lactide) (PLLA), poly(lactide-co-glycolide) (PLGA), pegylated PLGA, or a combination thereof.

In another specific embodiment, the coating comprises a hydrophobic polymer. In a more specific embodiment, the hydrophobic polymer comprises copolymers of styrene and isobutylene, polyorthoesters, polyanhydrides, or a combination thereof.

In certain embodiments, the polymer is biodegradable or biostable. In one embodiment, the polymer comprises excipients. In another embodiment, the polymer does not comprise excipients.

In a preferred embodiment, the medical device comprises a coating comprising a polymer, a free base form of halofuginone, and a salt form of halofuginone (*e.g*., hydrogen bromide, lactate). In another preferred embodiment, the medical device comprises a coating comprising a polymer, a first form of paclitaxel, and a second form of paclitaxel.

The medical device is suitable for insertion or implantation into a subject, preferably a human. Preferably, the medical device is a stent.

The invention also relates to methods of making the coated medical device. In certain embodiments, the method comprises providing a medical device having a surface suitable for exposure to the body tissue; and forming a coating on at least a portion of the surface, wherein the coating comprises a first polymer and a first form and a second form of a biologically active material.

In certain embodiments, the method comprise applying a coating composition to a medical device by spraying, dipping, direct deposition, or a combination thereof.

The invention further relates to methods for treating or preventing stenosis or restenosis or addressing other conditions (*e.g*., cancer) comprising inserting or implanting the medical device into a subject in need thereof. The medical device may be inserted or implanted alone or in combination with other treatment protocols.

### 4. FIGURES

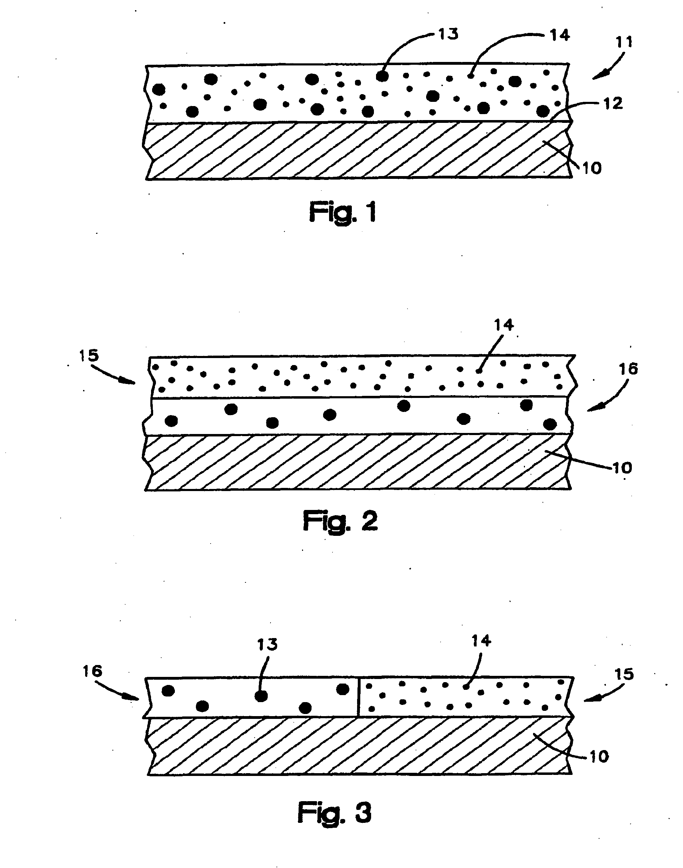

FIG. 1 shows a medical device **10** having a coating **11 on** a surface **12** as one embodiment of the present invention. The coating comprises a first form **13** and a second form **14** of a biologically active material.

FIG. 2 shows a medical device **10** having a coating **11 on** a surface **12** as one embodiment of the present invention. The coating **11** comprises (i) a first coating layer **15** comprising a first form **13** of a biologically active material, and (ii) a second coating layer **16** comprising a second form **14** of the biologically active material.

FIG. 3 shows a medical device **10** having a coating **11 on** a surface **12** as one embodiment of the present invention. A first portion **15** of the coating **11** comprises a first form **13** of a biologically active material, and a second portion **16** of the coating comprises a second form **14** of the biologically active material.

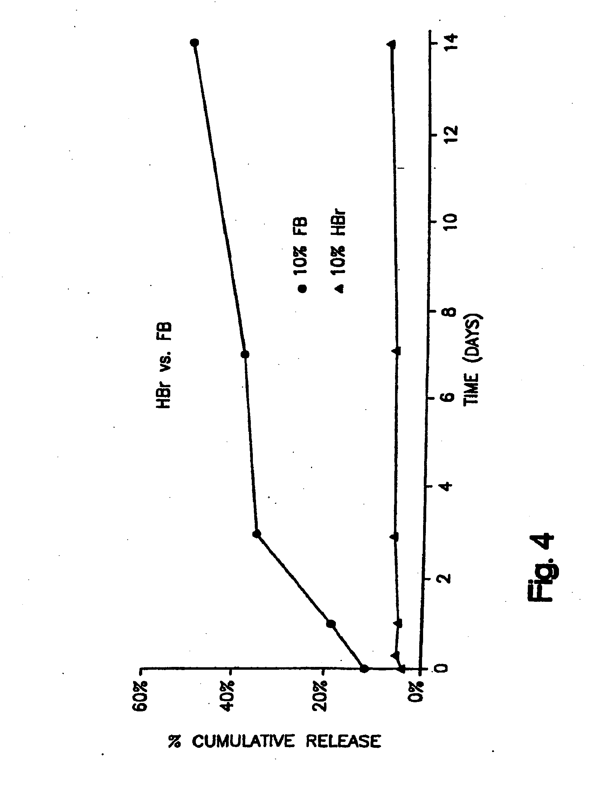

FIG. 4 shows the kinetic drug release (KDR) profile of a stent (HBr) coated with a hydrophobic polymer that comprises a hydrophilic form of a biologically active material and a stent (FB) coated with a hydrophobic polymer that comprises a hydrophobic form of the same biologically active material.

### 5. DETAILED DESCRIPTION OF THE INVENTION

The inventor has invented insertable or implantable drug-eluting medical devices comprising different forms of the same biologically active material in a coating **11.** Generally, the medical device **10** of the present invention has a coating **11** on a surface **12** of the medical device **10,** wherein the coating comprises two or more forms of the same biologically active material.

For clarity of disclosure, and not by way of limitation, the detailed description of the invention is divided into the subsections which follow.

### 5.1 COATED MEDICAL DEVICES

### 5.1.1 Methods for Preparing the Drug-Eluting Coating

Coating compositions suitable for forming the coating of the medical devices of the present invention can include one or more biologically active materials as describe in Section 5.1.1.1 *infra,* and one or more polymers as described in Section 5.1.1.2 *infra*. In one embodiment, the coating composition comprises a biologically active material that is present in at least two different forms.

To prepare the coating compositions, the constituents, *i.e*., polymer, biologically active material, and additional components, are suspended and/or dissolved in a solvent.

One or more solvents may be used with each coating composition. In one embodiment, the solvents used to prepare coating compositions include ones which can dissolve the polymeric material into solution or suspend the polymeric material. In another embodiment, the solvents used to prepare coating compositions include ones which can dissolve the polymeric material into solution or suspend the polymeric material. Any solvent which does not alter or adversely impact the therapeutic properties of the biologically active material can be employed.

The solvent in the coating composition can comprise one or more of the following solvents: tetrahydrofuran, chloroform, toluene, acetone, isooctane, 1,1,1-trichloroethane, or a mixture thereof. In addition to the solvent the polymer that is used in the coating composition can be styrene-isobutylene-styrene, polyurethanes, silicones, polyesters, polyolefins, polyisobutylene, ethylene-alphaolefin copolymers, acrylic polymers and copolymers, vinyl halide polymers, polyvinyl ethers, polyvinylidene halides, polyacrylonitrile, polyvinyl ketones, polyvinyl aromatics, polyvinyl esters, copolymers of vinyl monomers, copolymers of vinyl monomers and olefins, polyamides, alkyd resins, polycarbonates, polyoxymethylenes, polyimides, polyethers, epoxy resins, polyurethanes, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid, polyglycolic acid, polylactic acid-polyethylene oxide copolymers, EPDM rubbers, fluorosilicones, polyethylene glycol, polysaccharides, phospholipids, or a combination thereof.

In specific embodiments, a coating composition comprises at least 1%, at least 5%, at least 10%, at least 20%, at least 30%, at least 40%, at least 50%, at least 60%, at least 70%, at least 80%, at least 90%, at least 95%, at least 97%, at least 99% or more by weight of a polymer. In specific embodiments, a coating composition comprises at least 5%, at least 10%, at least 20%, at least 30%, at least 40%, at least 50%, at least 60%, at least 70%, at least 80%, at least 90%, at least 95%, at least 97%, at least 99% or more by weight of a biologically active material.

In certain embodiments, the coating composition comprises different polymer at different amounts or different ratios. In specific embodiments, a coating composition comprises a first polymer and a second polymer at a ratio of about 99:1, 95:5, 90:10, 80:20, 70:30, 60:40 or 50:50.

In certain embodiments, the coating composition comprises different biologically active materials at different amounts or different ratios. In specific embodiments, a coating composition comprises a first biologically active material and a second biologically active material at a ratio of about 99:1, 95:5, 90:10, 80:20, 70:30, 60:40 or 50:50.

In certain embodiments, the coating composition comprises different forms of a biologically active material at different amounts or different ratios. In specific embodiments, a coating composition comprises a first form and a second form of the biologically active material at a ratio of about 99:1, 95:5, 90:10, 80:20, 70:30, 60:40 or 50:50.

In certain embodiments, the coating is capable of providing sustained release of a biologically active material over a time period. In specific embodiments, the drug-eluting coating is capable of releasing about 1%, about 5%, about 10%, about 15%, about 20%, about 30%, about 40%, about 50%, about 60%, about 70%, about 80%, about 90% or more of the biologically active material over a time period. The time period for release of the biologically active material from the coating ranges from about 30 minutes, about 1 hour, about 2 hours, about 3 hours, about 4 hours, about 5 hours, about 6 hours, about 12 hours, about 1 day, about 2 days, about 3 days, about 4 days, about 5 days, about 6 days, about 1 week, about 2 weeks, about 3 weeks, about 1 month, about 2 months, about 3 months, about 4 months, about 5 months, about 6 months, about 1 year, about 2 years, or longer. Preferably, the time period for release of the biologically active material from the coating ranges from about 1 hour to about 24 months, preferably, from about 8 hours to about 9 months.

In a specific embodiment, the coating is capable of releasing one form of a biologically active material at a faster rate than the other form(s) of the biologically active material. Preferably, the coating is capable of releasing one form of a biologically active material at a rate that is about twenty times, about ten times, about five times, or about two times faster than the other form(s) of the biologically active material is being released. In another specific embodiment, the coating of the medical device is capable of releasing different forms of the biologically active material at about the same rate.

In another specific embodiment, the coating is capable of releasing more of one form of a biologically active material than the other form(s) of the biologically active material. Preferably, the coating is capable of releasing about twenty times, about ten times, about five times, or about two times more of one form of a biologically active material than the other form(s) of the biologically active material being released. In another specific embodiment, the coating is capable of releasing about the same amount of different forms of the biologically active material.

In certain embodiments, the coating comprises a plurality of coating layers, wherein each coating layer comprises one or more forms of the biologically active material. In one embodiment, the coating comprises a first coating layer that comprises a first form of a biologically active material, and a second coating layer that comprises a second form of the biologically active material. In one embodiment, the first coating layer is formed by applying a first coating composition comprising the first polymer and the first form of the biologically active material. In another embodiment, the second coating layer is formed by applying a second coating composition comprising a second polymer and the second form of the biologically active material. The coating may be formed by applying at least one coating composition by spraying, dipping, direct deposition, or a combination thereof, as described in Section 5.1.3 *infra*.

In a preferred embodiment, the first coating layer is substantially free of the second form of the biologically active material. In another preferred embodiment, the second coating layer is substantially free of the first form of the biologically active material. In yet another preferred embodiment, the first coating layer is substantially free of the second form of the biologically active material, and the second coating layer is substantially free of the first form of the biologically active material.

In a specific embodiment, the coating comprises the same amount/ratio of the different forms of the biologically active material. In another specific embodiment, the coating comprises different amounts/ratios of the different forms of the biologically active material.

In a solution not forming part of the invention the coating releases the different forms of the biologically active material in about the same amount, at about the same rate, and for about the same time period. In a specific embodiment, the coating releases the different forms of the biologically active material in different amounts, at different rates, and/or for different time periods.

In certain embodiments, the coating further comprises one or more polymers. Preferably, the two or more forms of the biologically active material are incorporated into a polymer. The cumulative release of the biologically active material from the polymer can be modulated by changing the relative amount of each form of the biologically active material within the polymer.

### 5.1.1.1 Biologically Active Material

In certain embodiments, the biologically active material is useful for inhibiting cell proliferation, contraction, migration, hyperactivity, or addressing other conditions such as cancer.

As used herein, the term "biologically active material" encompasses drugs, genetic materials, and biological materials. Non-limiting examples of suitable biologically active material include heparin, heparin derivatives, urokinase, dextrophenylalanine proline arginine chloromethylketone (PPack), enoxaprin, angiopeptin, hirudin, acetylsalicylic acid, tacrolimus, pimecrolimus, everolimus, rapamycin (sirolimus), amlodipine, doxazosin, glucocorticoids, betamethasone, dexamethasone, prednisolone, corticosterone, budesonide, sulfasalazine, rosiglitazone, mycophenolic acid, mesalamine, paclitaxel, 5-fluorouracil, cisplatin, vinblastine, vincristine, epothilones, methotrexate, azathioprine, adriamycin, mutamycin, endostatin, angiostatin, thymidine kinase inhibitors, cladribine, lidocaine, bupivacaine, ropivacaine, D-Phe-Pro-Arg chloromethyl ketone, platelet receptor antagonists, anti thrombin antibodies, anti platelet receptor antibodies, aspirin, dipyridamole, protamine, hirudin, prostaglandin inhibitors, platelet inhibitors, trapidil, liprostin, tick antiplatelet peptides, 5-azacytidine, vascular endothelial growth factors, growth factor receptors, transcriptional activators, translational promoters, antiproliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin, cholesterol lowering agents, vasodilating agents, agents which interfere with endogenous vasoactive mechanisms, antioxidants, probucol, antibiotic agents, penicillin, cefoxitin, oxacillin, tobranycin, angiogenic substances, fibroblast growth factors, estrogen, estradiol (E2), estriol (E3), 17-beta estradiol, digoxin, beta blockers, captopril, enalopril, statins, steroids, vitamins, taxol, paclitaxel, 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, 2'-O-ester with N-(dimethylaminoethyl) glutamide hydrochloride salt, nitroglycerin, nitrous oxides, nitric oxides, antibiotics, aspirins, digitalis, estrogen, estradiol and glycosides. In a preferred embodiment, the biologically active material is paclitaxel (*e.g*., Taxol®), or its analogs or derivatives. In yet another preferred embodiment, the biologically active material is an antibiotic such as erythromycin, amphotericin, rapamycin, adriamycin, etc. In yet another preferred embodiment, the biologically active material is halofuginone or a salt form of halofuginone including, but not limited to, hydrogen bromide, lactate, acetate, phosphate, and hydrogen chloride salts.

As used herein, the term "genetic materials" means DNA or RNA, including, without limitation, of DNA/RNA encoding a useful protein stated below, intended to be inserted into a human body including viral vectors and non-viral vectors.

As used herein, the term "biological materials" include cells, yeasts, bacteria, proteins, peptides, cytokines and hormones. Examples for peptides and proteins include vascular endothelial growth factor (VEGF), transforming growth factor (TGF), fibroblast growth factor (FGF), epidermal growth factor (EGF), cartilage growth factor (CGF), nerve growth factor (NGF), keratinocyte growth factor (KGF), skeletal growth factor (SGF), osteoblast-derived growth factor (BDGF), hepatocyte growth factor (HGF), insulin-like growth factor (IGF), cytokine growth factors (CGF), platelet-derived growth factor (PDGF), hypoxia inducible factor-1 (HIF-1), stem cell derived factor (SDF), stem cell factor (SCF), endothelial cell growth supplement (ECGS), granulocyte macrophage colony stimulating factor (GM-CSF), growth differentiation factor (GDF), integrin modulating factor (IMF), calmodulin (CaM), thymidine kinase (TK), tumor necrosis factor (TNF), growth hormone (GH), bone morphogenic protein (BMP) (*e.g*., BMP-2, BMP-3, BMP-4, BMP-5, BMP-6 (Vgr-1), BMP-7 (PO-1), BMP-8, BMP-9, BMP-10, BMP-11, BMP-12, BMP-14, BMP-15, BMP-16, etc.), matrix metalloproteinase (MMP), tissue inhibitor of matrix metalloproteinase (TIMP), cytokines, interleukin (*e.g*., IL-1, IL-2, IL-3, IL-4, IL-5, IL-6, IL-7, IL-8, IL-9, IL-10, IL-11, IL-12, IL-15, etc.), lymphokines, interferon, integrin, collagen (all types), elastin, fibrillins, fibronectin, vitronectin, laminin, glycosaminoglycans, proteoglycans, transferrin, cytotactin, cell binding domains (*e.g*., RGD), and tenascin. Currently preferred BMP's are BMP-2, BMP-3, BMP-4, BMP-5, BMP-6, BMP-7. These dimeric proteins can be provided as homodimers, heterodimers, or combinations thereof, alone or together with other molecules. Cells can be of human origin (autologous or allogeneic) or from an animal source (xenogeneic), genetically engineered, if desired, to deliver proteins of interest at the transplant site. The delivery media can be formulated as needed to maintain cell function and viability. Cells include progenitor cells (*e.g*., endothelial progenitor cells), stem cells (*e.g*., mesenchymal, hematopoietic, neuronal), stromal cells, parenchymal cells, undifferentiated cells, fibroblasts, macrophage, and satellite cells.

Other non-genetic biologically active materials include, but are not limited to:
- anti-thrombogenic agents such as heparin, heparin derivatives, urokinase, and PPack (dextrophenylalanine proline arginine chloromethylketone);
- anti-proliferative agents such as enoxaprin, angiopeptin, or monoclonal antibodies capable of blocking smooth muscle cell proliferation, hirudin, acetylsalicylic acid, tacrolimus, pimecrolimus, everolimus, amlodipine and doxazosin;
- anti-inflammatory agents such as glucocorticoids, betamethasone, dexamethasone, prednisolone, corticosterone, budesonide, estrogen, sulfasalazine, rosiglitazone, mycophenolic acid and mesalamine;
- anti-neoplastic/anti-proliferative/anti-miotic agents such as paclitaxel, 5-fluorouracil, cisplatin, vinblastine, vincristine, epothilones, methotrexate, azathioprine, adriamycin, mutamycin, endostatin, angiostatin, thymidine kinase inhibitors, cladribine, taxol and its analogs or derivatives;
- anesthetic agents such as lidocaine, bupivacaine, and ropivacaine;
- anti-coagulants such as D-Phe-Pro-Arg chloromethyl ketone, an RGD peptide-containing compound, heparin, antithrombin compounds, platelet receptor antagonists, antithrombin antibodies, anti-platelet receptor antibodies, aspirin (aspirin is also classified as an analgesic, antipyretic and anti-inflammatory drug), dipyridamole, protamine, hirudin, prostaglandin inhibitors, platelet inhibitors, antiplatelet agents such as trapidil or liprostin and tick antiplatelet peptides;
- DNA demethylating drugs such as 5-azacytidine, which is also categorized as a RNA or DNA metabolite that inhibit cell growth and induce apoptosis in certain cancer cells;
- vascular cell growth promoters such as growth factors, vascular endothelial growth factors (VEGF, all types including VEGF-2), growth factor receptors, transcriptional activators, and translational promoters;
- vascular cell growth inhibitors such as antiproliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin;
- cholesterol-lowering agents; vasodilating agents; and agents which interfere with endogenous vasoactive mechanisms;
- anti-oxidants, such as probucol;
- antibiotic agents, such as penicillin, cefoxitin, oxacillin, tobramycin, macrolides such as rapamycin (sirolimus) and everolimus;
- angiogenic substances, such as acidic and basic fibroblast growth factors, estrogen including estradiol (E2), estriol (E3) and 17-beta estradiol; and
- drugs for heart failure, such as digoxin, beta-blockers, angiotensin-converting enzyme (ACE) inhibitors including captopril and enalopril, statins and related compounds. Preferred biologically active materials include anti-proliferative drugs such as steroids, vitamins, and restenosis-inhibiting agents. Preferred restenosis-inhibiting agents include microtubule stabilizing agents such as Taxol®, paclitaxel (*i.e*., paclitaxel, paclitaxel analogues, or paclitaxel derivatives, and mixtures thereof). For example, derivatives suitable for use in the present invention include 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, and 2'-O-ester with N-(dimethylaminoethyl) glutamide hydrochloride salt.

Other preferred biologically active materials include nitroglycerin, nitrous oxides, nitric oxides, antibiotics, aspirins, digitalis, estrogen derivatives such as estradiol and glycosides.

The biologically active material can be present in a different forms, such as, but not limited to, a dispersion form or a solution form; a hydrophilic form or a hydrophobic form; a water soluble form or a water insoluble form; a lipid soluble form or a lipid insoluble form; a free acid form or a free base form or a salt form; an ionized form or a non-ionized form. The different forms of a biologically active material encompass the same biologically active material being at different physical and/or chemical states. The different forms of the biologically active material can occur naturally or be synthesized by any means known to one skilled in the art. These forms are described below.

In preferred embodiments, the biologically active material is present in a dispersion form or a solution form. In a preferred embodiment, the dispersion form of the biologically active material is water insoluble and/or lipid soluble. As used herein, the term "dispersion" refers to a mixture in which fine particles of one substance are scattered throughout another substance. In another preferred embodiment, the solution form of the biologically active material is water soluble and/or lipid insoluble. As used herein, the term "solution" refers to a homogeneous mixture of two or more substances.

In preferred embodiments, the biologically active material is present in a hydrophilic form or a hydrophobic form. In a preferred embodiment, the hydrophilic form of the biologically active material is water soluble and/or lipid insoluble. As used herein, the term "hydrophilic" refers to the characteristics of readily absorbing or dissolving in water, having polar groups (in which the distribution of electrons is uneven, enabling it to take part in electrostatic interactions) that readily interact with water, and/or having an affinity for water. In another preferred embodiment, the hydrophobic form of the biologically active material is water insoluble and/or lipid soluble. As used herein, the term "hydrophobic" refers to the characteristics of not readily absorbing or dissolving in water, being adversely effected by water, and/or having little or no affinity for water.

In preferred embodiments, the biologically active material is present in a free acid form or a free base form or a salt form. In a preferred embodiment, the biologically active material is present in a free acid form or a free base form. In another preferred embodiment, the biologically active material is present in a salt form. The free acid form and free base form of the biologically active material can be either water soluble or lipid soluble.

An acid is a substance which can donate a proton (H+ ion) to some other substance. The Arrhenius definition of an acid is a substance that when dissolved in water increases the concentration of hydrogen ions, H⁺*_{(αq)}.* A base is a substance which can accept a proton from other substances. The Arrhenius definition of a base is a substance that when added to water increases the concentration of hydroxide ion, OH⁻*_{(αq)}*.

The salt form of the biologically active material can be either water soluble or lipid soluble. A salt is formed between the reaction of an acid and a base. Usually a neutral salt is formed when a strong acid and a strong base is neutralized in a reaction. A salt that forms between a weak acid and a strong base is a basic salt (*e.g*., NaCH₃COO). A salt that forms between a strong acid and a weak base is an acid salt (*e.g*., NH₄Cl).

In specific embodiments, the biologically active material is present in an ionized form or a non-ionized form. In a preferred embodiment, the biologically active material is present in an ionized form. Preferably, the ionized form of the biologically active material is water soluble and/or lipid insoluble. As used herein, the term "ionized" means the gaining or losing of an electron. In another preferred embodiment, the biologically active material is present in a non-ionized form. Preferably, the non-ionized form of the biologically active material is water insoluble and/or lipid soluble.

In certain embodiments, the different forms of the biologically active materials for use in the medical devices of the present invention can be synthesized by methods well known to one skilled in the art. Alternatively, the different forms of the biologically active materials can be purchased from chemical and pharmaceutical companies.

In certain embodiments, the different forms of the biologically active material can be labelled with, *e.g*., radioisotopes, antibodies, or colored with, *e.g*., dye.

### 5.1.1.2 Polymer

As used herein, the term "polymer" is used interchangeable with the terms "polymer material" and "polymeric matrix".

The polymer suitable for use in the preparation of the drug-eluting coatings of the present invention should be a material that is biocompatible and avoids irritation to body tissue. Preferably, the polymer used in the coating compositions of the present invention are selected from the following: polyurethanes, silicones (*e.g*., polysiloxanes and substituted polysiloxanes), and polyesters. Also preferable as a polymeric material is copolymers of styrene and isobutylene, or more preferably, styrene-isobutylene-styrene (SIBS). In a specific embodiment, the polymeric material is a sulfonated SIBS, where "basic, + charged" compound capable of releasing NO are attached. Other polymers which can be used include ones that can be dissolved and cured or polymerized on the medical device or polymers having relatively low melting points that can be blended with biologically active materials. Additional suitable polymers include, thermoplastic elastomers in general, polyolefins, polyisobutylene, ethylene-alphaolefin copolymers, acrylic polymers and copolymers, vinyl halide polymers and copolymers such as poly(lactide-co-glycolide) (PLGA), polyvinyl alcohol (PVA), poly(L-lactide) (PLLA), polyanhydrides, polyphosphazenes, polycaprolactone (PCL), polyvinyl chloride, polyvinyl ethers such as polyvinyl methyl ether, polyvinylidene halides such as polyvinylidene fluoride and polyvinylidene chloride, polyacrylonitrile, polyvinyl ketones, polyvinyl aromatics such as polystyrene, polyvinyl esters such as polyvinyl acetate, copolymers of vinyl monomers, copolymers of vinyl monomers and olefins such as ethylene-methyl methacrylate copolymers, acrylonitrile-styrene copolymers, ABS (acrylonitrile-butadiene-styrene) resins, ethylene-vinyl acetate copolymers, polyamides such as Nylon 66 and polycaprolactone, alkyd resins, polycarbonates, polyoxymethylenes, polyimides, polyethers, epoxy resins, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid (PLA), polyglycolic acid (PGA), polyethylene oxide (PEO), polylactic acid-polyethylene oxide copolymers, EPDM (etylene-propylene-diene) rubbers, fluorosilicones, polyethylene glycol (PEG), polyalkylene glycol (PAG), polysaccharides, phospholipids, and combinations of the foregoing.

In certain embodiments, the polymer is hydrophilic (*e.g*., PVA, PLLA, PLGA, PEG, and PAG). In certain other embodiments, the polymer is hydrophobic (*e.g*., PLA, PGA, polyanhydrides, polyphosphazenes, PCL, copolymers of styrene and isobutylene, and polyorthoesters).

More preferably for medical devices which undergo mechanical challenges, *e.g*., expansion and contraction, the polymer should be selected from elastomeric polymers such as silicones (*e.g*., polysiloxanes and substituted polysiloxanes), polyurethanes, thermoplastic elastomers, ethylene vinyl acetate copolymers, polyolefin elastomers, and EPDM rubbers. Because of the elastic nature of these polymer, the coating composition is capable of undergoing deformation under the yield point when the device is subjected to forces, stress or mechanical challenge.

The polymer may be biodegradable or biostable. In preferred embodiments, the polymer is biodegradable. Biodegradable polymeric materials can degrade as a result of hydrolysis of the polymer chains into biologically acceptable, and progressively smaller compounds. In one embodiment, a polymeric material comprises polylactides, polyglycolides, or their co-polymers. Polylactides, polyglycolides, and their co-polymers break down to lactic acid and glycolic acid, which enters the Kreb's cycle and are further broken down into carbon dioxide and water.

Biodegradable solids may have differing modes of degradation. On one hand, degradation by bulk erosion/hydrolysis occurs when water penetrates the entire structure and degrades the entire structure simultaneously, *i.e*., the polymer degrades in a fairly uniform manner throughout the structure. On the other hand, degradation by surface erosion occurs when degradation begins from the exterior with little/no water penetration into the bulk of the structure (see, *e.g*., Gopferich A. Mechanisms of polymer degradation and erosion. Biomaterials 1996; 17(103):243-259. For some novel degradable polymers, most notably the polyanhydrides and polyorthoesters, the degradation occurs only at the surface of the polymer, resulting in a release rate that is proportional to the surface area of the drug delivery system. Hydrophilic polymeric materials such as PLGA will erode in a bulk fashion. Various commercially available PLGA may be used in the preparation of the coating compositions. For example, poly(d,1-lactic-co-glycolic acid) are commercially available. A preferred commercially available product is a 50:50 poly (D,L) lactic co-glycolic acid having a mole percent composition of 50% lactide and 50% glycolide. Other suitable commercially available products are 65:35 DL, 75:25 DL, 85:15 DL and poly(d,1-lactic acid) (d,1-PLA). For example, poly(lactide-co-glycolides) are also commercially available from Boehringer Ingelheim (Germany) under its Resomer©, *e.g*., PLGA 50:50 (Resomer RG 502), PLGA 75:25 (Resomer RG 728) and d,1-PLA (resomer RG 206), and from Birmingham Polymers (Birmingham, Alabama). These copolymers are available in a wide range of molecular weights and ratios of lactic to glycolic acid.

In one embodiment, the coating comprises copolymers with desirable hydrophilic/hydrophobic interactions (see, *e.g*., U.S. Patent No. 6,007,845, which describes nanoparticles and microparticles of non-linear hydrophilic-hydrophobic multiblock copolymers). In a specific embodiment, the coating comprises ABA triblock copolymers consisting of biodegradable A blocks from PLG and hydrophilic B blocks from PEO.

### 5.1.2 Types of Medical Devices

Medical devices that are useful in the present invention can be made of any biocompatible material suitable for medical devices in general which include without limitation natural polymers, synthetic polymers, ceramics, and metallics. In certain embodiments, ceramic material is preferred. Suitable ceramic materials include, but are not limited to, oxides, carbides, or nitrides of the transition elements such as titaniumoxides, hafnium oxides, iridiumoxides, chromium oxides, aluminum oxides, and zirconiumoxides. Silicon based materials, such as silica, may also be used. In certain other embodiments, metallic material (*e.g*., niobium, niobium-zirconium, and tantalum) is more preferable. Suitable metallic materials include metals and alloys based on titanium (such as nitinol, nickel titanium alloys, thermo-memory alloy materials), stainless steel, tantalum, nickel-chrome, or certain cobalt alloys including cobalt-chromium-nickel alloys such as Elgiloy® and Phynox®. Metallic materials also include clad composite filaments, such as those disclosed in WO 94/16646.

Metallic materials may be made into elongated members or wire-like elements and then woven to form a network of metal mesh. Polymer filaments may also be used together with the metallic elongated members or wire-like elements to form a network mesh. If the network is made of metal, the intersection may be welded, twisted, bent, glued, tied (with suture), heat sealed to one another; or connected in any manner known in the art.

The polymer(s) useful for forming the medical device should be ones that are biocompatible and avoid irritation to body tissue. They can be either biostable or bioabsorbable. Suitable polymeric materials include without limitation polyurethane and its copolymers, silicone and its copolymers, ethylene vinyl-acetate, polyethylene terephtalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polylactic acid, polyglycolic acid, polycaprolactone, polylactic acid-polyethylene oxide copolymers, cellulose, collagens, and chitins.

Other polymers that are useful as materials for medical devices include without limitation dacron polyester, poly(ethylene terephthalate), polycarbonate, polymethylmethacrylate, polypropylene, polyalkylene oxalates, polyvinylchloride, polyurethanes, polysiloxanes, nylons, poly(dimethyl siloxane), polycyanoacrylates, polyphosphazenes, poly(amino acids), ethylene glycol I dimethacrylate, poly(methyl methacrylate), poly(2-hydroxyethyl methacrylate), polytetrafluoroethylene poly(HEMA), polyhydroxyalkanoates, polytetrafluorethylene, polycarbonate, poly(glycolide-lactide) copolymer, polylactic acid, poly(ε-caprolactone), poly(β-hydroxybutyrate), polydioxanone, poly(γ-ethyl glutamate), polyiminocarbonates, poly(ortho ester), polyanhydrides, alginate, dextran, chitin, cotton, polyglycolic acid, polyurethane, or derivatized versions thereof, *i.e*., polymers which have been modified to include, for example, attachment sites or crosslinking groups, *e.g*., Arg-Gly-Asp (RGD), in which the polymers retain their structural integrity while allowing for attachment of molecules, such as proteins, nucleic acids, and the like.

The polymers may be dried to increase its mechanical strength. The polymers may then be used as the base material to form a whole or part of the medical device.

Furthermore, although the invention can be practiced by using a single type of polymer to form the medical device, various combinations of polymers can be employed. The appropriate mixture of polymers can be coordinated to produce desired effects when incorporated into a medical device.

In a specific embodiment, the medical device comprises a surface comprising a ceramic layer. Preferably, the ceramic layer extends the time period for releasing the biologically active material from the medical device.

The different forms of the biologically active material of the invention may also be used to form a medical or prosthetic device, preferably a stent, which may be inserted or implanted in a subject. In one embodiment, the different forms of the biologically active material of the invention may be incorporated into the base material needed to make the device. For example, in stent comprising a sidewall of elongated members or wire-like elements, the different forms of the biologically active material can be used to form the elongated members or wire-like elements.

In certain preferred embodiments, the different forms of the biologically active material described in Section 5.1.1 *supra.* are mixed with one or more polymers. Such mixture can be used to form a medical device or portions thereof. In specific embodiments, the biologically active material and/or coating compositions comprising the biologically active material constitute at least 1%, at least 5%, at least 10%, at least 20%, at least 30%, at least 40%, at least 50%, at least 60%, at least 70%, at least 80%, at least 90%, at least 95%, at least 97%, at least 99% or more by weight of the polymeric materials used to form the medical device.

Examples of the medical devices suitable for the present invention include, but are not limited to, stents, surgical staples, catheters (*e.g*., central venous catheters and arterial catheters), guidewires, cannulas, cardiac pacemaker leads or lead tips, cardiac defibrillator leads or lead tips, implantable vascular access ports, blood storage bags, blood tubing, vascular or other grafts, intra-aortic balloon pumps, heart valves, cardiovascular sutures, total artificial hearts and ventricular assist pumps, and extra-corporeal devices such as blood oxygenators, blood filters, hemodialysis units, hemoperfusion units and plasmapheresis units. In a preferred embodiment, the medical device is a stent.

Medical devices of the present invention include those that have a tubular or cylindrical-like portion. The tubular portion of the medical device need not to be completely cylindrical. For instance, the cross-section of the tubular portion can be any shape, such as rectangle, a triangle, etc., not just a circle. Such devices include, without limitation, stents and grafts. A bifurcated stent is also included among the medical devices which can be fabricated by the method of the present invention.

In addition, the tubular portion of the medical device may be a sidewall that is comprised of a plurality of struts defining a plurality of openings. The struts may be arranged in any suitable configuration. Also, the struts do not all have to have the same shape or geometric configuration. Each individual strut has a surface adapted for exposure to the body tissue of the patient. The tubular sidewall may be a stent.

In certain embodiments of the present invention, the insertable or implantable portion of the medical device of the present invention has a surface. The surface may have a plurality of openings therein. Preferably, the medical device is a stent having a sidewall comprising a plurality of struts defining a plurality of openings. When the medical device is a stent comprising a plurality of struts, the surface is located on the struts.

The medical device may be formed after application of the coating or it may be pre-fabricated before application of the coating. The pre-fabricated medical device is in its final shape. For example, if the finished medical device is a stent having an opening in its sidewall, then the opening is formed in the device before application of the coating.

Medical devices which are particularly suitable for the present invention include any kind of stent for medical purposes which is known to the skilled artisan. Suitable stents include, for example, vascular stents such as self-expanding stents and balloon expandable stents. Examples of self-expanding stents useful in the present invention are illustrated in U.S. Patent Nos. 4,655,771 and 4,954,11 issued to Wallsten and 5,061,275 issued to Wallsten et al*.* Examples of appropriate balloon-expandable stents are shown in U.S. Patent No. 5,449,373 issued to Pinchasik et al*.*

### 5.1.3 Methods of Coating the Medical Device

In the present invention, one or more coating compositions comprising the different forms of the biologically active material as described in Section 5.1.1.1 *supra.* can be applied by any method to a surface of a medical device to form a coating. Examples of suitable methods include, but are not limited to, spraying, laminating, pressing, brushing, swabbing, dipping, rolling, electrostatic deposition and all modem chemical ways of immobilization of bio-molecules to surfaces. Preferably, the coating composition is applied to a surface of a medical device by spraying, rolling, laminating, and pressing. In one embodiment of the present invention, more than one coating method can be used to make a medical device.

Furthermore, before applying the coating composition, the surface of the medical device is optionally subjected to a pre-treatment, such as roughening, oxidizing, sputtering, plasma-deposition or priming in embodiments where the surface to be coated does not comprise depressions. Sputtering is a deposition of atoms on the surface by removing the atom from the cathode by positive ion bombardment through a gas discharge. Also, exposing the surface of the device to a primer is a possible method of pre-treatment.

Multiple coating layers may be formed on the surface of the medical device. The coating layers may contain different materials, such as different polymers or different biologically active materials or different forms of a biologically active material, or each coating layer may contain the same combinations of polymers, but contain different amounts of each polymer. Alternatively, each coating layer may contain the same biologically active material but in different forms.

For example, a first coating layer and a second or additional coating layer may contain different materials that release certain biologically active materials or certain forms of the biologically active material at different rates. Also, the coating layers may be of different thicknesses and be arranged in any configuration on the medical device, such as disposed on different areas of the medical device or the first coating layer may cover the surface of the medical device and the second coating layer may be disposed on the first coating layer. For example, the coating layers may be adjacent on the surface of the medical device. Alternatively, a first coating layer may be disposed on the surface of the medical device and a second or additional coating layer may be disposed over at least a portion of the first coating layer. The second coating layer may or may not also be disposed on the surface of the medical device.

In certain embodiments, the coating of the medical device comprises a plurality of coating layers. In one embodiment, the coating of the medical device comprises (i) a first coating layer that comprises a first form of a biologically active material, and (ii) a second coating layer that comprises a second form of the biologically active material. In a specific embodiment, the first coating layer is substantially free of the second form of the biologically active material. In another specific embodiment, the second coating layer is substantially free of the first form of the biologically active material. In yet another embodiment, the first coating layer is substantially free of the second form of the biologically active material, and the second coating layer is substantially free of the first form of the biologically active material.

As shown in FIG. 1, in one embodiment of the present invention, a medical device comprises a surface and a coating disposed on the surface. The coating comprises a first form and a second form of a biologically active material. The coating may also contain one or more polymers.

As shown in FIG. 2, in one embodiment of the present invention, a medical device comprises a surface and a coating disposed on the surface. The coating comprises (i) a first coating layer comprising a first form of a biologically active material, and (ii) a second coating layer comprising a second form of the biologically active material.

As shown in FIG. 3, in another embodiment of the present invention, a medical device comprises a surface and a coating disposed on the surface. The coating includes two adjacent layers or portions. A first portion of the coating comprises a first form of a biologically active material, and a second portion of the coating comprises a second form of the biologically active material.

### 5.2 THERAPEUTIC USES

The invention relates generally to the therapeutic use of the coated medical devices made by the processes of Section 5.1 to address conditions such as stenosis, restenosis and cancer. Pharmaceutical compositions, body implants, and medical devices comprising the different forms of the biologically active material as described in Section *5.1.1.1 supra.* can be injected, inserted or implanted into a subject in need thereof.

In certain embodiments, the different forms of the biologically active material may be used to inhibit the proliferation, contraction, migration and/or hyperactivity of cells of the brain, neck, eye, mouth, throat, esophagus, chest, bone, ligament, cartilage, tendons, lung, colon, rectum, stomach, prostate, breast, ovaries, fallopian tubes, uterus, cervix, testicles or other reproductive organs, hair follicles, skin, diaphragm, thyroid, blood, muscles, bone, bone marrow, heart, lymph nodes, blood vessels, arteries, capillaries, large intestine, small intestine, kidney, liver, pancreas, brain, spinal cord, and the central nervous system. In a preferred embodiment, the biologically active material is useful for inhibiting the proliferation, contraction, migration and/or hyperactivity of muscle cells, *e.g*., smooth muscle cells.

In certain other embodiments, the biologically active material may be used to inhibit the proliferation, contraction, migration and/or hyperactivity of cells in body tissues, *e.g*., epithelial tissue, connective tissue, muscle tissue, and nerve tissue. Epithelial tissue covers or lines all body surfaces inside or outside the body. Examples of epithelial tissue include, but are not limited to, the skin, epithelium, dermis, and the mucosa and serosa that line the body cavity and internal organs, such as the heart, lung, liver, kidney, intestines, bladder, uterine, etc. Connective tissue is the most abundant and widely distributed of all tissues. Examples of connective tissue include, but are not limited to, vascular tissue (*e.g*., arteries, veins, capillaries), blood (*e.g*., red blood cells, platelets, white blood cells), lymph, fat, fibers, cartilage, ligaments, tendon, bone, teeth, omentum, peritoneum, mesentery, meniscus, conjunctiva, dura mater, umbilical cord, etc. Muscle tissue accounts for nearly one-third of the total body weight and consists of three distinct subtypes: striated (skeletal) muscle, smooth (visceral) muscle, and cardiac muscle. Examples of muscle tissue include, but are not limited to, myocardium (heart muscle), skeletal, intestinal wall, etc. The fourth primary type of tissue is nerve tissue. Nerve tissue is found in the brain, spinal cord, and accompanying nerve. Nerve tissue is composed of specialized cells called neurons (nerve cells) and neuroglial or glial cells.

The biologically active material, drug-eluting coatings, and coated medical devices of the present invention may also be used to treat diseases that may benefit from decreased cell proliferation, contraction, migration and/or hyperactivity, including, but not limited to stenosis, restenosis and cancer.

In particular, the biologically active material, such as paclitaxel, halofuginone, or a salt form of halofuginone, may be used to treat or prevent diseases or conditions that may benefit from decreased or slowed cell proliferation, contraction, migration or hyperactivity. In specific embodiments, the present invention inhibits or reduces at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, at least 10%, at least 5%, or at least 1% of cell proliferation, contraction, migration and/or hyperactivity.

The present invention further provides methods for treating or preventing cancer, stenosis or restenosis. In particular, the invention relates to methods for treating or preventing cancer, stenosis or restenosis by inserting or implanting a coated medical device of the invention into a subject.

As used herein, the terms "subject" and "patient" are used interchangeably. The subject can be an animal, preferably a mammal including a non-primate (*e.g*., a cow, pig, horse, cat, dog, rat, and mouse) and a primate (*e.g*., a monkey, such as a cynomolgous monkey, chimpanzee, and a human), and most preferably a human.

In one embodiment, the subject can be a subject who had undergone a regimen of treatment (*e.g*., percutaneous transluminal coronary angioplasty (PTCA), also known as balloon angioplasty, and coronary artery bypass graft (CABG) operation).

The therapeutically effective amount of a biologically active material for the subject will vary with the subject treated and the biologically active material itself. The therapeutically effective amount will also vary with the condition to be treated and the severity of the condition to be treated. The dose, and perhaps the dose frequency, can also vary according to the age, gender, body weight, and response of the individual subject. As used herein, the term "therapeutically effective amount" refers to that amount of the biologically active material sufficient to inhibit cell proliferation, contraction, migration, hyperactivity, or address other conditions (*e.g*., cancer). A therapeutically effective amount may refer to the amount of biologically active material sufficient to delay or minimize the onset of symptoms associated with cell proliferation, contraction, migration, hyperactivity, or address other conditions. A therapeutically effective amount may also refer to the amount of the biologically active material that provides a therapeutic benefit in the treatment or management of certain conditions such as cancer, stenosis or restenosis and/or the symptoms associated with cancer, stenosis or restenosis.

The present invention is useful alone or in combination with other treatment modalities. In certain embodiments, the subject can be receiving concurrently other therapies to treat or prevent cancer, stenosis or restenosis. In certain embodiments, the treatment of the present invention further includes the administration of one or more immunotherapeutic agents, such as antibodies and immunomodulators, which include, but are not limited to, HERCEPTIN®, RITUXAN®, OVAREX™, PANOREX®, BEC2, IMC-C225, VITAXIN™, CAMPATH® I/H, Smart MI95, LYMPHOCIDE™, Smart I D10, ONCOLYM™, rituximab, gemtuzumab, or trastuzumab. In certain other embodiments, the treatment method further comprises hormonal treatment. Hormonal therapeutic treatments comprise hormonal agonists, hormonal antagonists (*e.g*., flutamide, tamoxifen, leuprolide acetate (LUPRON™), LH-RH antagonists), inhibitors of hormone biosynthesis and processing, steroids (*e.g*., dexamethasone, retinoids, betamethasone, cortisol, cortisone, prednisone, dehydrotestosterone, glucocorticoids, mineralocorticoids, estrogen, testosterone, progestins), antigestagens (*e.g*., mifepristone, onapristone), and antiandrogens (*e.g*., cyproterone acetate).

### 6. EXAMPLES

### 6.1 STENTS COATED WITH A HYDROPHOBIC COATING COMPRISING EITHER A HYDROPHOBIC FORM OR HYDROPHILIC FORM OF HALOFUGINONE

### 6.1.1 Materials and Methods

The hydrophilic hydrogen bromide salt form of halofuginone was incorporated into a hydrophobic polymeric material using a suspension coating method. The hydrophilic form of the drug was discretely embedded in the hydrophobic polymeric material.

The hydrophobic free base form of the drug halofuginone was incorporated into a hydrophobic polymeric material using a solution coating method. The hydrophobic form of the drug was uniformly dispersed in the hydrophobic polymeric material.

A first stent (FB) was coated with the coating solution that comprises the hydrophobic (free base) form of halofuginone. A second stent (HBr) was coated with the coating solution that comprises the hydrophilic (hydrogen bromide salt) form of halofuginone. Both types of coating contain identical polymer to drug ratio (90:10, respectively), and therefore, the same amount of drug was loaded onto each stent.

The percentage of halofuginone released was measured for both stents.

### 6.1.2 Results

Although both stents were coated with the same amount of halofuginone, the two stents exhibited distinctive release profile of the drug eluting from the hydrophobic polymeric material (FIG. 4). Specifically, the FB stent (coated with a hydrophobic form of halofuginone) has a higher % cumulative release rate than the HBr stent (coated with a hydrophilic form of halofuginone). Also, a greater amount of halofuginone was released from the FB stent. In contrast, relatively little amount of halofuginone was released from the HBr stent.

The release rate and amount of halofuginone from the coating of the stents can be modulated by changing the relative amount of each form within the polymeric material.

## Claims

1. A coated medical device comprising:
a medical device having a surface suitable for exposure to a body tissue; and
a coating disposed on at least a portion of the surface, wherein the coating comprises (i) a first coating layer comprising a first polymer and a first form of a biologically active material and (ii) a second coating layer comprising a second polymer and a second form of the same biologically active material, wherein the coating releases the first form and the second form of the biologically active material at different rates.

2. The coated medical device of claim 1, wherein the first form is a dispersion form and the second form is a solution form, or wherein the first form is a hydrophilic form and the second form is a hydrophobic form, or wherein
the first form is a water soluble form and the second form is a water insoluble form, or wherein
the first form is a free acid form or free base form and the second form is a salt form, or wherein
the first form is an ionized form and the second form is a non-ionized form.

3. The coated medical device of claim 1, wherein the coating is capable of providing sustained release of the biologically active material over a time period from about 8 hours to 9 months.

4. The coated medical device of claim 1, wherein the biologically active material inhibits cell proliferation, contraction, migration or hyperactivity.

5. The coated medical device of claim 1, wherein the biologically active material comprises an immunosuppressant, an antiproliferative agent, or a combination thereof.

6. The coated medical device of claim 5, wherein
(a) the immunosuppressant comprises sirolimus, everolimus, tacrolimus, pimecrolimus, or a combination thereof, and
(b) the antiproliferative agent comprises paclitaxel.

7. The coated medical device of claim 1, wherein the biologically active material comprises halofuginone, or a salt form of halofuginone.

8. The coated medical device of claim 1, wherein the first polymer comprises styreneisobutylene-styrene, polyurethanes, silicones, polyesters, polyolefins, polyisobutylene, ethylene-alphaolefin copolymers, acrylic polymers and copolymers, vinyl halide polymers, polyvinyl ethers, polyvinylidene halides, polyacrylonitrile, polyvinyl ketones, polyvinyl aromatics, polyvinyl esters, copolymers of vinyl monomers, copolymers of vinyl monomers and olefins, polyamides, alkyd resins, polycarbonates, polyoxymethylenes, polyimides, polyethers, epoxy resins, polyurethanes, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid, polyglycolic acid, polylactic acid-polyethylene oxide copolymers, EPDM rubbers, fluorosilicones, polyethylene glycol, polysaccharides, phospholipids, or a combination thereof.

9. The coated medical device of claim 1, wherein the medical device is a stent.

10. The coated medical device of claim 1, wherein
(a) the first coating layer and the second coating layer comprise different polymers or combinations of polymers, or
(b) the first coating layer and the second coating layer each comprise the same polymer or combinations of polymers

11. The coated medical device of claim 1, wherein
(b) the first coating layer comprises the first polymer and a free base form of halofuginone, and
(c) the second coating layer comprises the second polymer and a salt form of halofuginone.

12. The coated medical device of claim 11, wherein the medical device is a stent.

13. The coated medical device of claim 1 wherein a plurality of coating layers are disposed on different parts of a surface of the medical device.

## Patentansprüche

1. Eine beschichtete medizinische Vorrichtung umfassend:
eine medizinische Vorrichtung mit einer Oberfläche geeignet zur Exposition in einem Körpergewebe; und
eine Beschichtung angelegt auf zumindest einem Teil der Oberfläche,
wobei die Beschichtung umfasst (i) eine erste Beschichtungsschicht umfassend ein erstes Polymer und eine erste Form von biologisch aktivem Material und (ii) eine zweite Beschichtungsschicht umfassend ein zweites Polymer und eine zweite Form desselben biologisch aktiven Materials, wobei die Beschichtung die erste Form und die zweite Form des biologisch aktiven Materials in verschiedenem Maß freisetzt.

2. Die beschichtete medizinische Vorrichtung nach Anspruch 1, wobei die erste Form eine Dispersionsform und die zweite Form eine Lösungsform ist, oder wobei
die erste Form eine hydrophile Form und die zweite Form eine hydrophobe Form ist, oder wobei
die erste Form eine wasserlösliche und die zweite Form eine wasserunlösliche Form ist, oder wobei
die erste Form eine freie Säureform oder freie Baseform und die zweite Form eine Salzform ist, oder wobei
die erste Form eine ionisierte Form ist und die zweite Form eine nichtionisierte Form ist.

3. Die beschichtete medizinische Vorrichtung nach Anspruch 1, wobei die Beschichtung geeignet ist eine fortgesetzte Freisetzung des biologisch aktiven Materials über eine Zeitspanne von etwa 8 Stunden bis 9 Monaten bereitzustellen.

4. Die beschichtete medizinische Vorrichtung nach Anspruch 1, wobei das biologisch aktive Material Zellproliferation, Kontraktion, Migration oder Hyperaktivität inhibiert.

5. Die beschichtete medizinische Vorrichtung nach Anspruch 1, wobei das biologisch aktive Material ein Immunsuppressivum, ein antiproliferativen Wirkstoff, oder eine Kombination davon umfasst.

6. Die beschichtete medizinische Vorrichtung nach Anspruch 5, wobei
(a) das Immunsuppressivum Sirolimus, Everolimus, Tacrolimus, Pimecrolimus, oder Kombinationen davon umfasst, und
(b) der antiproliferative Wirkstoff Paclitaxel umfasst.

7. Die beschichtete medizinische Vorrichtung nach Anspruch 1, wobei das biologisch aktive Material Halofuginon, oder eine Salzform von Halofuignon umfasst.

8. Die beschichtete medizinische Vorrichtung nach Anspruch 1, wobei das erste Polymer Styrenisobutylen-Styren, Polyurethane, Silikone, Polyester, Polyolefine, Polyisobutylene, Ethylen-Alphaolefin Copolymere, Akryl-Polymere und Copolymere, Vinylhalidpolymere, Polyvinylether, Polyvinylidenhalide, Polyakrylnitrile, Polyvinylketone, Polivinylaromaten, Polyvinylester, Copolymere der Vinylmonomere, Copolymere der Vinylmonomere und Olefine, Polyamide, Alkydharze, Polycarbonate, Polyoxymethylene, Polyimide, Polyether, Epoxyharze, Polyurethane, Rayon-Triacetate, Zellulose, Zelluloseacetate, Zellulosebutyrate, Zelluloseacetatbutyrate, Zellophane, Zellulosenitrate, Zellulosepropionate, Zelluloseether, Carboxymethylzellulose, Kollagene, Chitine, Polymilchsäuren, polyglykolische Säuren, Polymilchsäure-Polyethylenoxid-Copolymere, EPDM Gummi, Fluorosilikone, Polyethylenglykol, Polysaccharide, Phospholipide, oder eine Kombination davon umfasst.

9. Die beschichtete medizinische Vorrichtung nach Anspruch 1, wobei die medizinische Vorrichtung ein Stent ist.

10. Die beschichtete medizinische Vorrichtung nach Anspruch 1, wobei
(a) die erste Beschichtungsschicht und die zweite Beschichtungsschicht verschiedene Polymere und Kombinationen von Polymeren umfassen, oder
(b) die erste Beschichtungsschicht und die zweite Beschichtungsschicht jede das gleiche Polymer oder Kombinationen von Polymeren umfassen.

11. Die beschichtete medizinische Vorrichtung nach Anspruch 1, wobei
(b) die erste Beschichtungsschicht das erste Polymer und eine freie basische Form von Halofuginon umfasst, und
(c) die zweite Beschichtungsschicht das zweite Polymer und eine Salzform von Halofuginon umfasst.

12. Die beschichtete medizinische Vorrichtung nach Anspruch 11, wobei die medizinische Vorrichtung ein Stent ist.

13. Die beschichtete medizinische Vorrichtung nach Anspruch 1, wobei eine Vielzahl von Beschichtungsschichten auf verschiedenen Teilen der Oberfläche der medizinischen Vorrichtung angelegt sind.

## Revendications

1. Dispositif médical revêtu comportant :
un dispositif médical ayant une surface appropriée pour l'exposition à un tissu corporel ; et
un revêtement disposé sur au moins une portion de la surface, dans lequel le revêtement comporte (i) une première couche de revêtement comportant un premier polymère et une première forme d'un matériau biologiquement actif et (ii) une seconde couche de revêtement comportant un second polymère et une seconde forme du même matériau biologiquement actif, dans lequel le revêtement libère la première forme et
la seconde forme du matériau biologiquement actif à des cadences différentes.

2. Dispositif médical revêtu selon la revendication 1, dans lequel la première forme est une forme en dispersion et la seconde forme est une forme en solution, ou dans lequel la première forme est une forme hydrophile et la seconde forme est une forme hydrophobe, ou dans lequel
la première forme est une forme soluble dans l'eau et la seconde forme est une forme non soluble dans l'eau, ou dans lequel
la première forme est une forme d'acide libre et la seconde forme est une forme d'un sel, ou dans lequel
la première forme est une forme ionisée et la seconde forme est une forme non ionisée.

3. Dispositif médical revêtu selon la revendication 1, dans lequel le revêtement est apte à provoquer la libération soutenue du matériau biologiquement actif pendant une période de temps allant d'environ 8 heures à 9 mois.

4. Dispositif médical revêtu selon la revendication 1, dans lequel le matériau biologiquement actif inhibe la prolifération cellulaire, la contraction, la migration ou l'hyperactivité.

5. Dispositif médical revêtu selon la revendication 1, dans lequel le matériau biologiquement actif comprend un immunosuppresseur, un agent antiprolifératif, ou une combinaison de ceux-ci.

6. Dispositif médical revêtu selon la revendication 1, dans lequel
(a) l'immunosuppresseur comprend le sirolimus, l'évérolimus, le tacrolimus, le pimecrolimus, ou une combinaison de ceux-ci, et
(b) l'agent antiprolifératif comprend le paclitaxel.

7. Dispositif médical revêtu selon la revendication 1, dans lequel le matériau biologiquement actif comprend le halofuginone, ou une forme de sel de halofuginone.

8. Dispositif médical revêtu selon la revendication 1, dans lequel le premier polymère comprend le styrène-isobutylène-styrène, les polyuréthanes, les silicones, les polyesters, les polyoléfines, le polyisobutylène, les copolymères d'éthylène-alpha-oléfine, les polymères et copolymères acryliques, les polymères haloïdes de vinyle, les éthers de polyvinyles, les halogénures de polyvinylidène, le polyacrylonitrile, les cétones de polyvinyle, les composés aromatiques de polyvinyle, les esters de polyvinyle, les copolymères de monomères vinyliques, les copolymères de monomères vyniliques et d'oléfines, les polyamides, les résines alkydes, les polycarbonates, les polyoxyméthylènes, les polyimides, les polyéthers, les résines époxy, les polyuréthanes, la rayonne-triacétate, la cellulose, l'acétate de cellulose, le butyrate de cellulose, le butyrate d'acétate de cellulose, la cellophane, le nitrate de cellulose, le propionate de cellulose, les éthers de cellulose, la cellulose carboxyméthyl-cellulose, les collagènes, les chitines, l'acide polyactique, l'acide polyglyolique, les copolymères polyactiques d'oxyde d'acide-polyéthylène, les cacoutchoucs EPDM, les fluorosilicones, le polyéthylène glycol, les polysaccharides, les phospholipides, ou une combinaison de ceux-ci.

9. Dispositif médical revêtu selon la revendication 1, dans lequel le dispositif médical est un stent.

10. Dispositif médical revêtu selon la revendication 1, dans lequel
(a) la première couche de revêtement et la seconde couche de revêtement comportent différents polymères ou combinaisons de polymères, ou
(b) la première couche de revêtement et la seconde couche de revêtement comportent chacune le même polymère ou les mêmes combinaisons de polymères.

11. Dispositif médical revêtu selon la revendication 1, dans lequel
(b) la première couche de revêtement comporte le premier polymère et une forme basique libre de halofuginone, et
(c) la seconde couche de revêtement comporte le second polymère et une forme salée de halofuginone.

12. Dispositif médical revêtu selon la revendication 11, dans lequel le dispositif médical est un stent.

13. Dispositif médical revêtu selon la revendication 1, dans lequel une pluralité de couches de revêtement sont disposées sur différentes parties d'une surface du dispositif médical.
